(19) Europäisches Patentamt
European Patent Office
Office européen des brevets

(11) Publication number: **0 147 188 B1**

(12) # EUROPEAN PATENT SPECIFICATION

(45) Date of publication of patent specification: **03.08.88**

(51) Int. Cl.$^4$: **C 07 C 93/04,** C 07 D 295/08

(21) Application number: **84308954.1**

(22) Date of filing: **20.12.84**

(54) **Process for preparing tertiary polyalkylene aminoether alcohols.**

(30) Priority: **23.12.83 US 565098**

(43) Date of publication of application:
**03.07.85 Bulletin 85/27**

(45) Publication of the grant of the patent:
**03.08.88 Bulletin 88/31**

(84) Designated Contracting States:
**BE DE FR GB IT NL**

(56) References cited:
**EP-A-0 006 454**
**EP-A-0 084 943**
**EP-A-0 087 856**
**DE-A-2 009 918**
**FR-A-2 404 636**
**US-A-3 654 370**
**US-A-4 487 967**

(73) Proprietor: **Exxon Research and Engineering Company**
**P.O.Box 390 180 Park Avenue**
**Florham Park New Jersey 07932 (US)**

(72) Inventor: **Ho, Win-sow Winston**
**15 Nottingham Road Annandale**
**New Jersey 08801 (US)**
Inventor: **Montagna, Angelo Anthony**
**Fairway View, Apts. 2245, College Drive, Apt. 173**
**Baton Rouge Louisiana 70808 (US)**
Inventor: **Stogryn, Eugene Leo**
**3 Sharp Road Edison**
**New Jersey 08817 (US)**

(74) Representative: **Somers, Harold Arnold et al**
**ESSO Engineering (Europe) Ltd.**
**Patents & Licences**
**Apex Tower High Street**
**New Malden Surrey KT3 4DJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Field of the Invention

The present invention relates to a process for selectively preparing tertiary polyalkylene aminoether alcohols, some of which are severely sterically hindered, by reacting an acyclic or heterocyclic amino compound with a polyalkylen ether glycol in the presence of a hydrogenation catalyst at elevated temperatures and pressures.

Background of the Invention

Recently, it has been disclosed that tertiary aminoether alcohols are suitable agents for scrubbing acid gases from gaseous streams containing the same. Such compositions are disclosed in BE—A—769,797 as well as in Goodridge, *Trans Faraday Society, 51,* 1703—9 (1955). The art teaches that these compounds may be prepared by reacting the appropriate secondary amine or heterocyclic nitrogeneous compound with a haloalkoxyalkane or sulfonate ester of a glycolic ether under conditions such that the haloacid or sulfonic acid is eliminated. Disadvantages of such a process includes relatively expensive raw materials and product isolation. That is, the initially formed product is an amine salt which requires caustic treatment as part of the product isolation procedure.

Consequently, there exists a need in the art for methods of preparing such tertiary aminoether alcohols which would not be limited by such disadvantages.

Summary of the Invention

In accordance with the present invention there is provided a process for preparing cyclic and severely sterically hindered acyclic tertiary polyalkylen aminoether alcohols which comprises reacting

(a) one or more secondary acyclic or heterocyclic amino compounds respectively represented by the formulas:

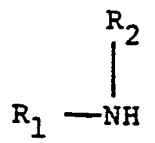

and

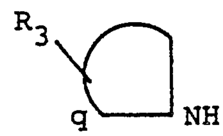

where $R_1$ and $R_2$ are independently selected from the group consisting of primary, secondary and tertiary alkyl radicals having 1 to 8 carbon atoms and cycloalkyl radicals having 3 to 8 carbon atoms with the proviso that the least one of $R_1$ and $R_2$ is a secondary or tertiary alkyl radical, q is the number, from 2 to 10, of carbon atoms in the heterocyclic ring, and $R_3$, which is optional, is selected from the group consisting of alkyl and cycloalkyl radicals which may be pending on one or more of the carbon atoms of the ring; with

(b) a polyalkylen ether glycol having the general formula:

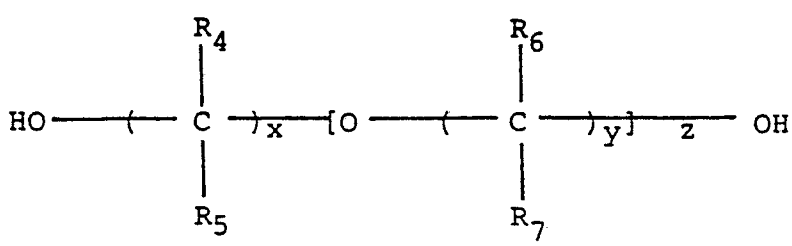

where $R_4$, $R_5$, $R_6$, and $R_7$ are each independently selected from the group consisting of hydrogen $C_1$ to $C_4$ alkyl radicals, and $C_3$—$C_8$ cycloalkyl radicals with the proviso that if only one of $R_1$ and $R_2$ directly attached to the nitrogen atom is a secondary alkyl radical and neither of them is a tertiary alkyl radical, at least one of $R_4$ and $R_5$ directly bonded to the carbon which is bonded to the hydroxyl group is an alkyl or cycloalkyl radical, x and y are each positive integers independently ranging from 2 to 4, and z is a positive integer ranging from 1 to 10, said process being carried out in the presence of a catalytically effective amount of a supported Group VIII metal-containing hydrogenation catalyst at elevated temperatures and pressures, wherein the mole ratio of amino compound employed to polyalkylen ether glycol is less than 2:1 when z is greater than 1.

In preferred embodiments of the present invention, $R_1$ is a tertiary alkyl radical having 4 to 6 carbon atoms, $R_2$ is an alkyl radical having 1 to 6 carbon atoms, $R_4$, $R_5$, $R_6$, and $R_7$ are hydrogen, x and y are 2 and z is from 1 to 10.

In other preferred embodiments of the present invention, the amino compound is heterocyclic, preferably having 3 to 6 carbon atoms in the ring, $R_4$, $R_5$, $R_6$, and $R_7$ are hydrogen, x and y are 2 and z is from 1 to 10.

Detailed Description of the Invention

As previously indicated, both secondary acyclic amino compounds and heterocyclic amino compounds may be used in the practice of the present invention. The preferred amino compound is N-methyl-tertiary-butyl amine and the preferred heterocyclic amino compounds are saturated and contain from 3 to 6 carbon atoms in the ring, more preferably 4 or 5 carbon atoms, and most perferably 4 carbon atoms.

The amination process hereof is carried out under pressure at a temperature ranging from 160° to 425°C, preferably from 180° to 400°C, and most preferably from 190° to 350°C. The (gauge) pressure in the reactor may range from 50 to 3000 psig, (345 to 20685 kPa), preferably from 100 to 1000 psig (689 to 6895 kPa), and most preferably from 150 to 750 psig (1034 to 5171 kPa).

The reactor used may include any suitable vessel capable of withstanding the pressures necessary to carry out the amination process. Preferably, the anination process is carried out in a fixed bed reactor whereby the reactants are passed over a fixed bed of the catalyst, either concurrently or countercurrently. Other reactors suitable for use herein include moving bed reactors and continuous stirred reactors. For example, in a continuous stirred reactor the catalyst is circulated and the reactants and reaction product are passed through the reaction vessel at a controlled rate.

The hydrogenation catalyst used in the amination process herein may include any of the known hydrogenation catalysts. Illustrative hydrogenation catalysts include platinum, palladium and other noble metals such as ruthenium, rhodium, osmium and iridium deposited on inert supports such as carbon, silica, alumina or other refractory oxides or inert supports such as carbon, silica, alumina or other refractory oxides, Raney nickel, nickel-on-kieselguhr, nickel on inert support, massive nickel or nickel-cobalt or nickel-cobalt-copper coprecipitated with silicate and/or aluminum salts having alumina or kieselguhr supports. Preferred catalysts include coprecipitated nickel, nickel-cobalt, and nickel-cobalt-copper supported on silica, alumina or a mixture thereof. Also referred is platinum supported on alumina. Still more preferred are catalysts having increasing concentations of nickel, about 40% to 70% nickel, by weight. Since preferred catalysts include those massive-metal coprecipitated hydrogenation catalysts described in U.S. Patent Nos. 3,697,445; 4,251,394; 4,251,672; 4,263,173; 4,263,225; 4,273,680; 4,273,939; 4,307,248; 4,318,829; and the metal coprecipitated catalysts containing aluminum and silica disclosed and claimed in U.S. Serial Nos. 388,966 and 388,967 corresponding to EP—A—0097047. It is preferred that the catalyst be reduced or activated by a reductant, such as hydrogen prior to use in the amination reaction. This reduction or activation is typically carried out by passing hydrogen over the catalyst at temperatures ranging from 175° to 400°C, preferably 200° to 340°C.

The concentration of the hydrognation catalyst is that which is catalytically effective and that amount will generally range from 0.1 to 10 weight percent, preferably from 2 to 8 weight percent, based on the weight of the reactant charge. The normal pretreatment conditions and handling of the hydrogenation catalyst should be practiced as known to those skilled in the hydrogenation catalyst art.

The theoretical equivalent mole ratio of amino compound to polyalkylen ether glycol in the reaction charge is 1:1. When the polyalkylen ether glycol is diethylene glycol the mole ratio of amino compound to diethylene glycol can range from 0.5:1 to 6:1, preferably 1:1 to 4:1. When the polyalkylen ether glycol is triethylene glycol or higher, the mole ratio of amino compound to glycol must be kept below 2:1 otherwise the tertiary amino ether alcohol would not be favored.

For purposes of this invention it may be desirable to include an inert solvent in the reaction medium. Preferably the solvent is a solvent such as cyclic or linear ether or a hydrocarbon-containing compound in which the reactants will dissolve. The solvent should be of relatively low molecular weight to facilitate its removal from the product of the reaction. The amount of the solvent may vary, but will generally range from 10 to 50 wt.% preferably from 15 to 30 wt.%, based on the weight of the reactants used. Preferred solvents include tetrahydrofuran, dimethylether of ethylene glycol and toluene.

Reduction of the catalyst may be carried out in situ while conducting the process by the presence of hydrogen. Hydrogen, however, is not essential to conducting the process but is preferably employed, for example, to minimize catalyst deactivation.

Once the reaction has been completed, the reaction product can be conveniently recovered by known techniques such as solvent evaporation, distillation and the like.

The invention is illustrated further by the following paper examples which, however, are not to be taken as limiting in any respect. All parts are percentages, unless expressly stated otherwise, are by weight.

### Example 1

N-Ethoxyethanol Pyrrolidine

A mixture of 20g of pyrrolidine, 29g of diethylene glycol, 50ml of toluene and 6g of 1% platinum supported on graphite are heated at 200°C in a 300ml stirred steel autoclave at autogenous pressure. At the end of 24 hrs N-ethoxyethanol pyrrolidine will be obtained in a yield of about 40%.

### Example 2

N-Methyl-tertiarybutylaminoethoxyethanol

A 300ml steel autoclave containing .5 moles of N-methyl-tertiarylamine, .5 mole of diethylene glycol and 1g of a supported nicket catalyst (Harshaw—Ni—5132P), are pressured with 100 psi (689.5 kPa) of

hydrogen and heated at 200°C until the diethylene glycol is completely consumed. Both the disappearance of diethylene glycol and the formation of N-methyl-tertiarybutylaminoethoxyethanol will be followed by *the periodic withdrawal of samples from the autoclave, followed by G. C. analysis.*

## Claims

1. A process for producing tertiary polyalkylene aminoether alcohols comprising reacting:

(a) one or more secondary acyclic or heterocyclic amino compounds respectively represented by the formulas:

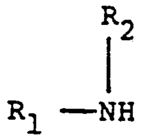

and

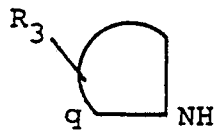

where $R_1$ and $R_2$ are independently selected from the group consisting of primary, secondary and tertiary alkyl radicals having 1 to 8 carbon atoms and cycloalkyl radicals having 3 to 8 carbon atoms with the proviso that the least one of $R_1$ and $R_2$ is a secondary or tertiary alkyl radical, q is the number, from 2 to 10, of carbon atoms in the heterocyclic ring, and $R_3$, which is optional, is selected from the group consisting of alkyl and cycloalkyl radicals which may be pendent on one or more of the carbon atoms of the ring; with

(b) a polyalkylene ether glycol having the general formula:

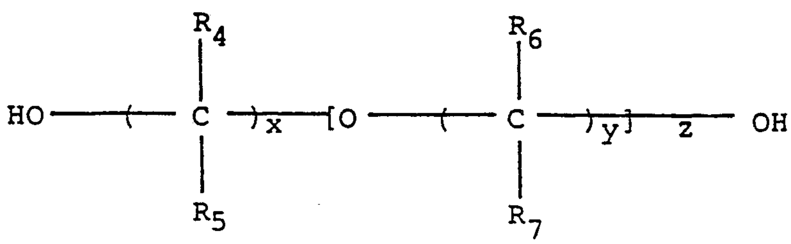

where $R_4$, $R_5$, $R_6$, and $R_7$ are each independently selected from the group consisting of hydrogen, $C_1$ to $C_4$ alkyl radicals, and $C_3$—$C_8$ cycloalkyl radicals with the proviso that if only one of $R_1$ and $R_2$ directly attached to the nitrogen atom is a secondary alkyl radical and neither of them is a tertiary alkyl radical, at least one of $R_4$ and $R_5$ directly bonded to the carbon which is bonded to the hydroxyl group is an alkyl or cycloalkyl radical, x and y are each positive integers independently ranging from 2 to 4, and z is a positive integer ranging from 1 to 10, said process being carried out in the presence of a catalytically effective amount of a supported Group VIII metal-containing hydrogenation catalyst at elevated temperatures and pressures, and wherein the mole ratio of amino compound employed to polyalkylene ether glycol is less than 2:1 when z is greater than 1.

2. The process of claim 1 wherein $R_1$ is an alkyl radical having 4 to 6 carbon atoms, $R_2$ is an alkyl radical having 1 to 6 carbon atoms, $R_4$, $R_5$, $R_6$ and $R_7$ are hydrogen, and x and y are both 2.

3. The process of either one of the preceeding claims wherein $R_1$ is tertiary butyl, $R_2$ is methyl, and z is 1.

4. The process of any one of the preceding claims wherein the amino compound is a heterocyclic compound having 3 to 6 carbon atoms in the ring $R_4$, $R_5$, $R_6$ and $R_7$ are hydrogen, and x and y are both 2.

5. The process of any one of the preceding claims wherein the heterocyclic compound has 4 to 5 carbon atoms in the ring and z is 1.

6. The process of any one of the preceding claims wherein the reaction is carried out at a temperature ranging from 160° to 425°C, and at a (gauge) pressure ranging from 50 to 3000 psig (344.75 to 20685 kPa).

7. The process of any one of the preceding claims wherein the reaction is carried out at a temperature ranging from 180° to 400°C, and at a (gauge) pressure ranging from about 100 to about 1000 psig (689.5 to 6895 kPa).

8. The process of any one of the preceding claims wherein the reaction is carried out at a temperature ranging from 190°C to 350°C and at a (gauge) pressure ranging from 150 psig to 750 psig (1034 to 5171 kPa).

9. The process of any one of the preceding claims wherein the hydrogenation catalyst is a nickel, a cobalt, a nickel-cobalt, a nickel-cobalt-copper, or a platinum catalyst all of which are supported on alumina silica or a combination thereof.

**Patentansprüche**

1. Verfahren zur Herstellung tertiärer Polyalkylenaminoetheralkohole durch Umsetzung von:

(a) einer oder mehreren sekundären acyclischen oder heterocyclischen Aminoverbindungen der allgemeinen Formeln:

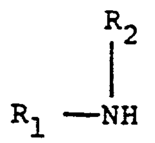 bzw. 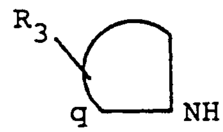

worin $R_1$ und $R_2$ aus der Gruppe bestehend aus primären, sekundären und tertiären Alkylresten mit 1 bis 8 Kohlenstoffatomen und Cycloalkylresten mit bis 8 Kohlenstoffatomen mit der Maßgabe ausgewählt sind, daß zumindest einer von $R_1$ und $R_2$ ein sekundärer oder tertiärer Alkylrest ist, q von 2 bis 10 ist und die Anzahl der Kohlenstoffatome in dem heterocylischen Ring angibt und der gegebenenfalls vorhandene Rest $R_3$ aus der Gruppe bestehend aus Alkyl- und Cycloalkylresten ausgewählt ist, die an ein oder mehrere der Kohlenstoffatome des Rings gebunden sein können; mit

(b) einem Polyalklenetherglykol der allgemeinen Formel:

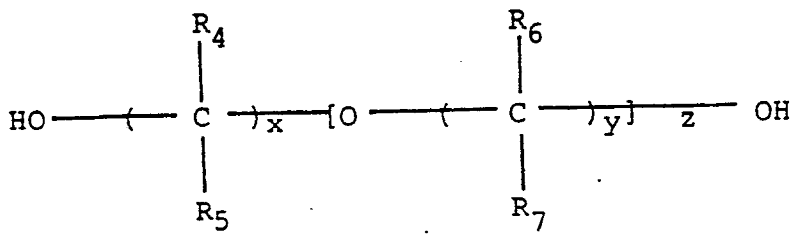

in der $R_4$, $R_5$, $R_6$ und $R_7$ jeweils unabhängig voneinander aus der Gruppe bestehend aus Wasserstoff, $C_1C_4$-Alkylresten und $C_3$—$C_8$ Cycloalkylresten imit der Maßgabe ausgewählt sind, daß wenn nur einer der direkt an das Stickstoffatom gebundenen $R_1$ und $R_2$ ein sekundärer Alkylrest und keiner von beiden ein tertiärer Alkylrest ist, zumindest einer der direkt an das Kohlenstoffatom, welches and die Hydroxylgruppe gebunden ist, gebundenen $R_4$ und $R_5$ ein Alkyl-oder Cycloalkylrest ist, x and y jeweils unabhängig voneinander positive ganze Zahlen im Bereich von 2 bis 4 sind und z eine positive ganze Zahl im Bereich von 1 bis 10 ist, wobei das Verfahren bei erhöhten Temperaturen und Drücken in Gegenwart einer katalytisch wirksamen Menge eines ein Gruppe-VIII Metall enthaltenden Hydrierungs-Trägerkatalysators durchgeführt wird und wobei das molare Verhältnis von Aminoverbindung zu Polyalkylenetherglykol kleiner als 2:1 ist, wenn z größer als 1 ist.

2. Verbunden nach Anspruch 1, in dem $R_1$ ein Alkylrest mit 4 bis 6 Kohlenstofftomen ist, $R_2$ ein Alkylrest mit 1 bis 6 Kohlenstofftomen ist, $R_4$, $R_5$, $R_6$ und $R_7$ Wasserstoff sind x und y beide 2 sind.

3. Verfahren nach einem der vorangehenden Ansprüche, in dem $R_1$ tert.-Butyl ist, $R_2$ Methyl ist und z 1 ist.

4. Verfahren nach einem der vorangehenden Ansprüche, in dem die Aminoverbindung eine heterocyclische Verbindung mit 3 bis 6 Kohlenstoffatomen in dem Ring ist, $R_4$, $R_5$, $R_6$ und $R_7$ Wasserstoff sind x und y beide 2 sind.

5. Verfahren nach einem der vorangehenden Ansprüche, in dem die heterocyclische Verbindung 4 bis 5 Kohlenstoffatome in dem Ring aufweist und z 1 ist.

6. Verfahren nach einem der vorangehenden Ansprüche, in dem die Reaktion bei einer Temperatur im Bereich von 160 bis 425°C und bei einem Überdruck im Bereich von 344,75 bis 20685 kPa durchgeführt wird.

7. Verfahren nach einem der vorangehenden Ansprüche, in dem die Reaktion bei einer Temperatur im Bereich von 180 bis 400°C und bei einem Überdruck im Bereich von 689,5 bis 6895 kPa durchgeführt wird.

8. Verfahren nach einem der vorangehenden Ansprüche, in dem die Reaktion bei einer Temperatur im Bereich von 190 bis 350°C und bei einem Überdruck im Bereich von 1034 bis 5171 kPa durchgeführt wird.

9. Verfahren nach einem der vorangehenden Ansprüche, in dem der Hydrierungskatalysator ein Nickel-, ein Kobalt-, ein Nickel-Kobalt-, ein Nickel-Kobalt-Kupfer- oder ein Platin-Katalysator ist, wobei alle auf Aluminiumoxid, Siliciumdioxid oder einer Mischung davon aufgebracht sind.

**Revendications**

1. Procédé de production de polyalkylène-aminoéther-alcools tertiaires, comprenant la réaction

(a) d'un ou plusieurs composés amino acycliques ou hétérocycliques secondaires, respectivement représentés par les formules:

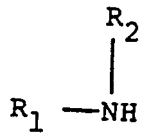

et

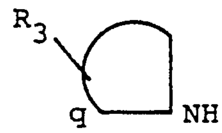

dans lesquelles $R_1$ et $R_2$ sont choisis dans le groupe constitué par des radicaux alkyle primaires, secondaires et tertiaires ayant 1 à 8 atomes de carbone et des radicaux cycloalkyle ayant 3 à 8 atomes de carbone, à condition que l'un au moins de $R_1$ et $R_2$ soit un radical alkyle secondaire ou tertiaire, q est le nombre d'atomes de carbone, de 2 à 10, dans le noyau hétérocyclique, et $R_3$, qui est facultatif, est choisi dans le groupe constitué par des radicaux alkyle et cycloalkyle qui peuvent être pendants sur un ou plusieurs des atomes de carbone du noyau; avec

(b) un polyalkylène-éther-glycol ayant la formule générale:

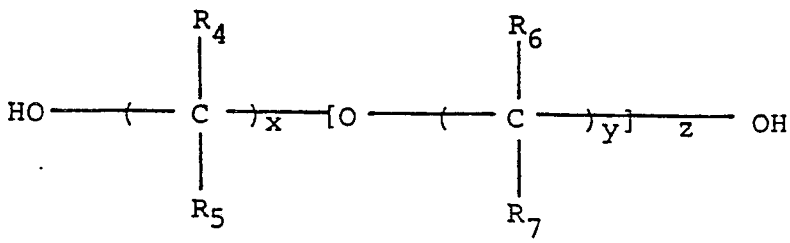

dans laquelle $R_4$, $R_5$, $R_6$ et $R_7$ sont chacun choisis indépendamment dans le groupe constitué par un hydrogène, des radicaux alkyle en $C_1$ à $C_4$ et des radicaux cycloalkyle en $C_3$—$C_8$, à condition que, si l'un seulement de $R_1$ et $R_2$, directement fixés à l'atome d'azote, est un radical alkyle secondiare et qu'aucun d'entre eux n'est un radical alkyle tertiaire, l'un au moins de $R_4$ et $R_5$, directement fixés au carbone qui est fixé au groupe hydroxyle, soit un radical alkyle ou cycloalkyle, x et y sont chacun des nombres entiers positifs valant indépendamment de 2 à 4, et z est un nombre entier positif valant de 1 à 10, ce procédé étant réalisé en présence d'une quantité catalytiquement efficace d'un catalyseur d'hydrogénation supporté contenant un métal du groupe VIII, à des températures et sous des pressions élevées, et dans lequel le rapport molaire du composé amino au polyalkylène-èther-glycol est inférieur à 2:1 lorsque z est supérieur à 1.

2. Procédé selon la revendication 1, dans lequel $R_1$ est un radical alkyle ayant 4 à 6 atomes de carbone, $R_2$ est un radical alkyle ayant 1 à 6 atomes de carbone, $R_4$, $R_5$, $R_6$ et $R_7$ sont des atomes d'hydrogène et x et y sont tous deux égaux à 2.

3. Procédé selon l'une ou l'autre des revendications précédentes, dans lequel $R^1$ est un tertiobutyle, $R_2$ est un méthyle et z est égal à 1.

4. Procédé selon l'une quelconque des revendications précédentes, dans lequel le composé amino est un composé hétérocyclique ayant 3 à 6 atomes de carbone dans le noyau, $R_4$, $R_5$, $R_6$ et $R_7$ sont des atomes d'hydrogène et x et y sont tous deux égaux à 2.

5. Procédé selon l'une quelconque des revendications précédentes, dans lequel le composé hétérocyclique a 4 à 5 atomes de carbone dans le noyau et z est égal à 1.

6. Procédé selon l'une quelconque des revendications précédentes, dans lequel la réaction est réalisée à une température allant de 160° à 425°C et sous une pression manométrique allant de 50 à 3 000 psig (344,75 à 20 685 kPa).

7. Procédé selon l'une quelconque des revendications précédentes, dans lequel la réaction est réalisée à une température allant de 180° à 400°C et sous une pression manométrique allant d'environ 100 à environ 1 000 psig (689,55 à 6 895 kPa).

8. Procédé selon l'une quelconque des revendications précédentes, dans lequel la réaction est réalisée à une température allant de 190° à 350°C et sous une pression manométrique allant de 150 à 750 psig (1 034 à 5 171 kPa).

9. Procédé selon l'une quelconque des revendications précédentes, dans lequel le catalyseur d'hydrogénation est un catalyseur au nickel, au cobalt, au nickel-cobalt, au nickel-cobalt-cuivre ou au platine, supporté à chaque fois sur de l'alumine, de la silice ou une combinaison des deux.